# EUROPEAN PATENT APPLICATION

(11) **EP 4 785 810 A1**
(43) Date of publication of application: **05.08.2026**
(21) Application number: 24872508.7
(22) Date of filing: 27.09.2024
(51) Int. Cl.: A23L 33/10, A61K 8/9706, A61K 8/9722, A61K 36/02, A61K 36/05, A61P 17/00, A61P 43/00, A61Q 19/00, C12N 1/12

(54) **COMPOSITION FOR MAINTAINING OR INCREASING FIBROUS STRUCTURAL PROTEIN**

(30) Priority: 29.09.2023 JP 2023170831
(71) Applicant: Rohto Pharmaceutical Co., Ltd, Osaka-shi Osaka 544-8666 (JP)
(72) Inventor: YAMADA,Kotaro, Osaka-shi, Osaka 544-8666 (JP); SOEJIMA,Yoshiomi, Osaka-shi, Osaka 544-8666 (JP); NAKAHARA,Ken, Osaka-shi, Osaka 544-8666 (JP); TAIRA,Toshio, Osaka-shi, Osaka 544-8666 (JP); MATSUMOTO,Nozomi, Osaka-shi, Osaka 544-8666 (JP); MIZUTANI,Kaoru, Osaka-shi, Osaka 544-8666 (JP)
(74) Representative: Schön, Christoph
(86) International application number: PCT/JP2024/034653
(87) International publication number: WO 2025/070726

(57) **Abstract**

An object of the present invention is to provide a formulation effective for maintaining or increasing hair fibrous structural proteins. Provided are a composition for maintaining or increasing fibrous structural proteins, a composition for maintaining or increasing skin elasticity, a composition for improving hair quality, and a composition for promoting production of collagen, elastin, or hyaluronic acid, which contain a culture supernatant and/or extracellular vesicles of a microalga.

## Description

### TECHNICAL FIELD

The present invention relates to a composition for maintaining or increasing fibrous structural proteins.

### BACKGROUND ART

Skin is roughly divided into epidermis and dermis. In dermis, the reticular layer is a layer that makes up the majority of dermis, where there are fibers and a matrix, called the extracellular matrix (Extracellular matrix, ECM). Examples of the fibrous structural proteins in the ECM include fiber components such as collagen and elastin, and substrate components such as hyaluronic acid.

Elastin is composed of a filamentous protein in the form of a yellow random coil, and is known to have a strong elasticity like rubber that returns to its original state even when stretched nearly twice and then relaxed. Elastin crosslinks collagen in the connective tissue to support the collagen like a spring, which becomes a motive force of expansion and contraction, and greatly functions to maintain elasticity, firmness, and the like of skin. In vivo, elastin is widely distributed in stretchable organs such as dermis of skin, ligaments, tendons, and blood vessel walls, and plays a very important role in providing elasticity in these organs.

However, it is known that the amount of elastin is gradually reduced due to aging, ultraviolet rays, active oxygen, stress, and the like. It has been found that this reduction gradually loses elasticity that should originally exist, causes wrinkles and sagging on skin and the like, and becomes a major cause of aging.

It has also been previously known that deficiency of normal elastin can contribute to vascular diseases such as arteriosclerosis.

Hitherto, several elastin production promoters have been proposed to improve the condition due to elastin reduction. For example, in Patent Document 1, an extract of Polygonatum plant is proposed as an elastin production promoter.

In the dermal extracellular matrix of skin, collagen forms a reticulated bundle to maintain the tissue morphology. When collagen matures and proliferates, and crosslinking proceeds, the collagen becomes a thick linear fiber bundle to provide moderate firmness on young skin. However, in aged skin, collagen in the dermal extracellular matrix is significantly reduced, so that original firmness having rich elasticity is lost. As a result, wrinkles and sagging are formed on skin. The change in collagen fiber bundle structure of a hairless mouse due to photoaging has been studied in detail (Non-Patent Document 1), and it has been shown that wrinkles are formed on a hairless mouse irradiated with UVB, and the collagen fiber bundle structure is disintegrated so as to coincide with the formation of the wrinkles, so that skin elasticity decreases. Collagen is also known to have an excellent moisture-retaining function.

For example, in Non-Patent Document 2, retinoic acid is proposed as a collagen production promoter.

Hyaluronic acid is a mucopolysaccharide composed of glucuronic acid and N-acetylglucosamine, which is a substance that is present in a large amount in dermis in vivo as a bonding substance filling spaces among cells or fibers and is confirmed to be present also in epidermis, and has high water retainability and viscoelasticity (Non-Patent Document 3).

### PRIOR ART DOCUMENT

### PATENT DOCUMENT

Patent Document 1: JP-W-2001-513509

### NON-PATENT DOCUMENTS

Non-Patent Document 1: Fragrance Journal, No. 4, p36-37, 1998
Non-Patent Document 2: R. Marks et al., British Journal of Dermatology, 122, 91-98, 1990
Non-Patent Document 3: Fragrance Journal, No. 2, 30, 1990

### SUMMARY OF THE INVENTION

### PROBLEMS TO BE SOLVED BY THE INVENTION

As described above, it is presumed that a component capable of maintaining or increasing fibrous structural proteins such as collagen, elastin, and hyaluronic acid leads to maintenance or improvement of the skin condition, but what kind of component is suitable from the viewpoint of maintaining or increasing hair fibrous structural proteins has not been sufficiently reported yet.

Accordingly, it is an object of the present invention to provide a composition effective for maintaining or increasing fibrous structural proteins.

### MEANS FOR SOLVING THE PROBLEMS

In order to solve the above problems, the present inventors have extensively conducted studies, and resultantly found that a culture supernatant and/or extracellular vesicles of a microalga have an action on maintenance or increase of fibrous structural proteins, thereby completing the present invention.

That is, a preferred embodiment of the present invention provides the following composition.

[1] A composition for maintaining or increasing a fibrous structural protein, containing a culture supernatant and/or extracellular vesicles of a microalga.

### EFFECT OF THE INVENTION

According to the present invention, it is possible to maintain and increase fibrous structural proteins by using a culture supernatant and/or extracellular vesicles of a microalga.

### BRIEF DESCRIPTION OF THE DRAWINGS

Fig. 1 is a graph showing a nanoparticle analysis result of a Pavlova culture supernatant ultrafiltrated sample in Test Example 1.
Fig. 2 is a graph showing a nanoparticle analysis result of a Spirulina culture supernatant ultrafiltrated sample in Test Example 1.
Fig. 3 is a graph showing a nanoparticle analysis result of a Chlorella vulgaris culture supernatant ultrafiltrated sample in Test Example 1.
Fig. 4 is a graph showing results of a test for adding a culture supernatant and/or extracellular vesicles of a microalga to human fibroblasts in Test Example 2.
Fig. 5 is a graph showing results of a test for adding a culture supernatant and/or extracellular vesicles of a microalga to human fibroblasts in Test Example 2.
Fig. 6 is a graph showing results of a test for adding a culture supernatant and/or extracellular vesicles of a microalga to human fibroblasts in Test Example 2.
Fig. 7 is a graph showing results of a test for adding a culture supernatant and/or extracellular vesicles of a microalga to human fibroblasts in Test Example 2.
Fig. 8 is a graph showing results of a test for adding a culture supernatant and/or extracellular vesicles of a microalga to human fibroblasts in Test Example 2.
Fig. 9 is a graph showing results of a test for adding a culture supernatant and/or extracellular vesicles of a microalga to human fibroblasts in Test Example 2.
Fig. 10 is a graph showing results of a test for adding a culture supernatant and/or extracellular vesicles of a microalga to human fibroblasts in Test Example 2.
Fig. 11 is a graph showing results of a test for adding a culture supernatant and/or extracellular vesicles of a microalga to three-dimensional cultured skin in Test Example 3.
Fig. 12 is a graph showing results of a test for adding a culture supernatant and/or extracellular vesicles of a microalga to three-dimensional cultured skin in Test Example 3.
Fig. 13 is a graph showing results of a test for adding a culture supernatant and/or extracellular vesicles of a microalga to three-dimensional cultured skin in Test Example 3.

### MODE FOR CARRYING OUT THE INVENTION

### [Composition for maintaining or increasing fibrous structural protein]

In one embodiment, the composition for maintaining or increasing fibrous structural proteins of the present invention contains a culture supernatant and/or extracellular vesicles of a microalga.

### (Culture supernatant and/or extracellular vesicles of microalga)

In the present description, "microalgae" refer to organisms having a cell size of 0.1 µm to 1000 µm in diameter among the rest of organisms performing photosynthesis other than mosses, ferns, and seed plants that generate oxygen.

Examples of the microalgae include organisms of prokaryotes such as cyanobacteria, and eukaryotes such as Glaucophyta, Rhodophyta (red algae), Chlorophyta, Cryptophyta, Haptophyta, Heterokontophyta, Dinophyta, Euglenida, and Chlorarachniophyta.

Examples of the microalgae include microalgae belonging to the genus Pavlova, Euglena (Protozoa/green algae), Spirulina (blue-green algae), Chlorella (green algae), Dunaliella (green algae), Nannochloropsis (eustigmatophyceans), Scenedesmus (green algae), or Botryococcus (green algae). The microalgae exemplified above may be used alone or in combination of two or more thereof.

Among the microalgae, from the viewpoint of remarkably exhibiting the effect of the present invention, microalgae belonging to at least one species selected from the group consisting of the genus Pavlova, Euglena, Spirulina, and Chlorella are preferable, microalgae belonging to at least one species selected from the group consisting of the genus Pavlova, Euglena, and Spirulina are more preferable, microalgae belonging to at least one species selected from the group consisting of the genus Pavlova and Euglena are still more preferable, and microalgae belonging to the genus Pavlova are particularly preferable.

Pavlova is included in the phylum of the Haptophyta, and examples of the microalgae of the genus Pavlova include P. calceolate, P. granifera, P. gyrans, P. lutheri, P. pinguis, and P. salina. Among them, P. granifera and/or P. gyrans are preferable.

Examples of the microalgae of the genus Euglena include E. gracilis, E. longa, E. caudata, E. oxyuris, E. tripteris, E. proxima, E. viridis, E. sociabilis, E. ehrenbergii, E. deses, E. pisciformis, E. spirogyra, E. acus, E. geniculata, E. intermedia, E. mutabilis, E. sanguinea, E. stellata, E. terricola, E. klebsi, E. rubra, and E. cyclopicola. Among them, E. gracilis and/or E. longa are preferable.

The microalgae of the genus Spirulina may be, for example, the genus Arthrospira, a scientific name changed from the genus Spirulina, and examples thereof include S. platensis (A. platensis) and S. maxima (A. maxima). Among them, S. platensis is preferable.

Examples of the microalgae of the genus Chlorella include C. vulgaris, C. saccharophila, C. Ellipsoidea, C. pyrenoidosa, C. sorokiniana, and C. lobophora. Among them, C. vulgaris is preferable.

The microalgae exemplified above are widely distributed in sea water, fresh water such as in ponds and marshes, and brackish water. The microalgae may be separated from the water and then used. Alternatively, any microalgae already isolated may be used.

The microalgae exemplified above include related species and mutant strains thereof as long as the effect of the present invention is exhibited. Examples of the mutant strain include those obtained by a genetic method such as genetic recombination, transduction, or transformation.

In the present description, the culture supernatant of a microalga refers to a supernatant of a culture solution after the microalga is cultured. Examples of the supernatant include a supernatant of a culture solution in which a microalga has been cultured by a known culture method and from which the microalga has been removed by a known separation means such as centrifugation. Here, the culture supernatant preferably contains extracellular vesicles derived from a microalga.

Culture conditions for preparing the culture supernatant of a microalga are not particularly limited as long as the effect of the present invention is exhibited, and a known method can be used. The following examples are given.

### (Culture conditions)

The microalga can be cultured using a culture solution. To the culture solution, a carbon source is added as a nutrient source, and as the carbon source, an inorganic carbon source (CO₂, NaHCO₃, Na₂CO₃, etc.), an organic carbon source (glucose or the like), or the like is used. The culture solution is not limited, but an autotrophic medium not containing an organic carbon source such as glucose as a nutrient source is preferably used. Examples of the culture solution include a culture solution to which a nutrient salt such as a nitrogen source, a phosphorus source, or a mineral is added, such as a Cramer-Myers medium or a modified Cramer-Myers medium.

When sea water is used depending on the type of microalgae, a culture medium for marine microalgae, artificial sea water, or the like can also be used, or a commercially available product such as an IMK culture medium can also be used.

The pH of the culture solution is not particularly limited as long as the effect of the present invention is exhibited, and examples thereof include 2 to 8, 3 to 8, 4 to 8, 5 to 8, 2 to 7.5, 3 to 7.5, 4 to 7.5, and 5 to 7.5.

The culture temperature is not particularly limited as long as the effect of the present invention is exhibited, and examples thereof include 15 to 40°C, 20 to 34°C, and 23 to 28°C.

The culture period is not particularly limited as long as the effect of the present invention is exhibited, and examples thereof include 4 to 30 days, 4 to 20 days, and 5 to 15 days.

Depending on the type of microalgae, the type of light source, the presence or absence of light and darkness, aerobic conditions, anaerobic conditions, and the like can be appropriately selected.

In one embodiment, in the case of culturing a marine microalga such as Pavlova, a known method can be used as long as the effect of the present invention is exhibited, and for example, culturing can be performed under conditions satisfying at least one of the following conditions:
Seawater concentration: 50% seawater
Medium concentration: IMK medium 2-fold concentration
Amount of culture solution: 800 mL
Light source: Side surface: Fluorescent lamp (100 to 150 µmol)
Light/dark cycle: 12 hours each for light and dark
pH: about pH 7.4 at the start of each culture
Aeration: done
Mechanical stirring: not done
Culture period: done for a period of about 10 days.
Culture temperature: 25°C to 28°C.

### (Collection of culture supernatant)

After the microalga is cultured under the culture condition exemplified above, the culture supernatant is collected using a known separation means such as centrifugation, but the collection method is not particularly limited as long as the culture supernatant of the microalga can be appropriately collected.

In the present invention, extracellular vesicles of a microalga can also be used. In the present description, the extracellular vesicle refers to a vesicle having an average diameter of usually 10 nm to 1 µm and secreted from a microalga. Although not limited, when extracellular vesicles of a microalga are used in the composition of the present invention, it is preferable that cells themselves of a microalga be not contained.

The method for separating extracellular vesicles is not particularly limited, and a known separation means can be used. As the method for separating extracellular vesicles, for example, a technique such as centrifugation can be used, but the method is not limited thereto.

In one embodiment, the method for producing extracellular vesicles includes, but is not limited to, a method including (1) a step of centrifuging a culture supernatant of a microalga to yield a supernatant; and (2) a step of further centrifuging the yielded product as necessary to yield a precipitate; by which microalga-derived extracellular vesicles that exhibit an effective activity can be produced.

In an exemplary embodiment, the centrifugation in said steps (1) and (2) may be carried out for 40 to 80 minutes or 30 to 60 minutes at a centrifugal force of, for example, 1,000 to 20,000 × g, 1,500 to 20,000 × g, 1,500 to 15,000 × g, or 1,500 to 10,000 × g. At this time, the centrifugation may be carried out stepwise by changing a speed or time. For example, the centrifugation may be performed at a low speed of 1,500 to 2,000 × g to separate a culture supernatant, followed by centrifugation at a high speed of 10,000 to 20,000 × g to further remove cells or cell-associated remnants, residues, and the like.

As a method for producing extracellular vesicles, a method can also be used in which a culture supernatant of a microalga is filtered through a filter. The membrane pore diameter of the filter for use in the filtration is, for example, preferably 0.001 µm to 0.2 µm, more preferably 0.005 µm to 0.1 µm, still more preferably 0.01 µm to 0.05 µm.

Here, the filter may be in a filter form performing filtration through a hollow fiber membrane from the outside to the inside of the fiber. Alternatively, the filter may be a spiral membrane including a filtration membrane and a support membrane, a tubular membrane on a hollow cylinder, or a flat membrane. Among them, a hollow fiber membrane is preferably used.

The hollow fiber membrane is a form of a permeable membrane, and refers to a membrane body formed in a thin straw-like thin tube. In the hollow fiber membrane module, a hollow fiber membrane bundle obtained by bundling a large number of the hollow fiber membranes is stored in a sealed state in a housing container formed of a synthetic resin of vinyl chloride or a metal such as aluminum. In general, the diameter (inner diameter) per hollow fiber membrane is about 100 µm to several mm, but is preferably 100 µm to 800 µm, more preferably 200 µm to 600 µm for efficiently circulating a liquid. The length of each hollow fiber membrane can be adjusted depending on the application, but when the length is too long, the pressure for flowing a liquid increases, so that the hollow fiber membrane is formed in the form of a hollow fiber membrane bundle with a length excluding the fixing portions at both ends, that is, a so-called effective length of preferably 120 mm to 450 mm, more preferably 100 mm to 300 mm, still more preferably 150 mm to 250 mm. From the viewpoint of industrialization, the length is preferably 250 mm to 1000 mm, more preferably 400 mm to 800 mm. The membrane thickness of the hollow fiber membrane is generally about 10 µm to 100 µm, but is preferably 20 µm to 60 µm, more preferably 20 µm to 50 µm, still more preferably 30 µm to 50 µm. In the present invention, from the viewpoint of purification efficiency, it is preferable to use the hollow fiber membrane module (the hollow fiber membrane constitutes the hollow fiber membrane module).

The membrane area of the hollow fiber membrane in the hollow fiber membrane module for use in the present invention can be a converted value at the intermediate portion of the thickness, that is, an average value of the area of the outer surface portion and the area of the inner surface portion, in consideration of the diameter difference between the inside and the outside of the hollow fiber membrane. Since the membrane area and the treatment speed of the hollow fiber membrane are in a proportional relationship, the use of a hollow fiber membrane module having a larger membrane area increases the treatment speed, so that the time required for treatment can be shortened, and the damage visited on extracellular vesicles can be reduced. On the other hand, since the membrane area of the hollow fiber module and the dead volume (residual amount in the flow path) during purification are also in a proportional relationship, when a hollow fiber module having a larger membrane area is used, the loss increases. Accordingly, it is preferable to select a hollow fiber membrane module having an appropriate membrane area depending on the purpose. The membrane area of the hollow fiber membrane in the hollow fiber membrane module for use in the present invention is appropriately selected from the range of about 1 cm² to 3,000 cm² depending on the purpose. For example, in a case where it is intended to reduce the volume by concentration, it is preferable to use a module having a small membrane area of about 20cm². In addition, in a case where the purpose is concentration of the cell culture supernatant, filtration, removal of impurities, and the like, it is preferable to use a hollow fiber membrane module having a membrane area of, for example, about 115 cm², about 1,000 cm², or about 1,600 cm² depending on the treatment amount. From the viewpoint of industrialization, the area of the hollow fiber membrane in the hollow fiber module is preferably 1,000 cm² to 100,000 cm², more preferably 10,000 cm² to 60,000 cm².

The pore diameter of the hollow fiber membrane (including the hollow fiber membrane provided in the hollow fiber membrane module) for use in the present invention can be selected depending on the purpose. For example, when impurities larger than desired extracellular vesicles are removed, a pore diameter that does not allow only the impurities to permeate is selected. Such a pore diameter is preferably 0.001 to 0.2 µm, more preferably 0.005 µm to 0.1 µm, still more preferably 0.01 µm to 0.05 µm. The pore diameter may also be 0.0001 µm or more, 0.0005 µm or more, 0.001 µm or more, 0.005 µm or more, 0.01 µm or more, 0.015 µm or more, 0.2 µm or less, 0.15 µm or less, 0.1 µm or less, 0.05 µm or less, 0.04 µm or less, or 0.03 µm or less.

Cutoff membranes having a molecular weight cutoff of 10 kDa to 1,000 kDa, 50 kDa to 1000 kDa, and 100 kDa to 1,000 kDa are preferably used, and a cutoff membrane having a molecular weight cutoff of 100 kDa to 500 kDa is more preferably used. Cutoff membranes are preferable having a molecular weight cutoff of 10 kDa or more, 50 kDa or more, 100 kDa or more, and 150 kDa or more, and also preferable having a molecular weight cutoff of 2000 kDa or less, 1500 kDa or less, 1000 kDa or less, 750 Dka or less, and 500 DKa or less.

After culturing a microalga, by continuously diluting and concentrating the culture supernatant or the like after removing the cells, unnecessary substances are removed, and at the same time, solvent exchange is performed, whereby necessary extracellular vesicles can be obtained.

The material of the hollow fiber membrane provided in the filter, especially the hollow fiber membrane module, is not particularly limited as long as the material is a material that can be used for the purpose of appropriately separating extracellular vesicles derived from cells, and examples thereof include polyethersulfone (PES), modified polyethersulfone (mPES), and polyacrylonitrile (PAN). Among them, polyethersulfone (PES) and modified polyethersulfone (mPES) are preferable, and modified polyethersulfone (mPES) is more preferable, from the viewpoint of high hydrophilicity and low protein adsorptivity.

The extracellular vesicles of the present invention can also be obtained by heating a culture supernatant or a culture of a microalga under a temperature condition of, for example, but not limited to, 40°C or higher, 50°C or higher, 60°C or higher, 70°C or higher, 80°C or higher, 90°C or higher, 100°C or higher, 200°C or lower, 150°C or lower, or 120°C or lower in a time condition of 30 seconds or longer, 1 minute or longer, 2 minutes or longer, 3 minutes or longer, 4 minutes or longer, 30 minutes or shorter, 20 minutes or shorter, or 10 minutes or shorter.

The extracellular vesicles of the present invention can also be obtained by a method including heating a culture supernatant or a culture of a microalga and concentrating or purifying the heated culture supernatant or culture through filtration with a filter.

The present invention includes extracellular vesicles obtained from a culture supernatant of a microalga. The extracellular vesicles can also be obtained as a concentrate or a purified solution (extracellular vesicle-containing substance) obtained by filtering the culture supernatant with a filter. The extracellular vesicle-containing substance may be in the form of a concentrated solution or a purified solution itself of a culture supernatant, but may be a solid or a semi-solid obtained by drying the concentrated solution or the purified solution.

The extracellular vesicles of the present invention can also be made into a population having high purity of the extracellular vesicles through further optional purification.

The extracellular vesicles of the present invention may be in the form of a concentrated solution or a purified solution itself of a culture supernatant, but may be a solid or a semi-solid obtained by drying the concentrated solution or the purified solution.

In the composition of the present invention, examples of the average number of particles of extracellular vesicles of a microalga include 1.0 × 10³ or more, 1.0 × 10⁴ or more, 1.0 × 10⁵ or more, 1.0 × 10⁶ or more, 1.0 × 10⁷ or more, 1.0 × 10⁸ or more, or 1.0 × 10⁹ or more, and 1.0 × 10¹⁴ or less, 1.0 × 10¹³ or less, 1.0 × 10¹² or less, 1.0 × 10¹¹ or less, and 1.0 × 10¹⁰ or less. The average number of particles of extracellular vesicles of a microalga is, for example, 1.0 × 10⁴ to 1.0 × 10¹⁴, 1.0 × 10⁴ to 1.0 × 10¹³, 1.0 × 10⁴ to 1.0 × 10¹², 1.0 × 10⁴ to 1.0 × 10¹¹, 1.0 × 10⁴ to 1.0 × 10¹⁰, 1.0 × 10⁵ to 1.0 × 10¹⁴, 1.0 × 10⁵ to 1.0 × 10¹³, 1.0 × 10⁵ to 1.0 × 10¹², 1.0 × 10⁵ to 1.0 × 10¹¹, 1.0 × 10⁵ to 1.0 × 10¹⁰, 1.0 × 10⁶ to 1.0 × 10¹⁴, 1.0 × 10⁶ to 1.0 × 10¹³, 1.0 × 10⁶ to 1.0 × 10¹², 1.0 × 10⁶ to 1.0 × 10¹¹, 1.0 × 10⁶ to 1.0 × 10¹⁰, 1.0 × 10⁷ to 1.0 × 10¹⁴, 1.0 × 10⁷ to 1.0 × 10¹³, 1.0 × 10⁷ to 1.0 × 10¹², 1.0 × 10⁷ to 1.0 × 10¹¹, 1.0 × 10⁷ to 1.0 × 10¹⁰, 1.0 × 10⁸ to 1.0 × 10¹⁴, 1.0 × 10⁸ to 1.0 × 10¹³, 1.0 × 10⁸ to 1.0 × 10¹², 1.0 × 10⁸ to 1.0 × 10¹¹, 1.0 × 10⁸ to 1.0 × 10¹⁰, 1.0 × 10⁹ to 1.0 × 10¹⁴, 1.0 × 10⁹ to 1.0 × 10¹³, 1.0 × 10⁹ to 1.0 × 10¹², 1.0 × 10⁹ to 1.0 × 10¹¹, or 1.0 × 10⁹ to 1.0 × 10¹⁰. For counting the number of extracellular vesicles, an exosome measurement system (ExoCounter, JVC Kenwood Corporation) by a sandwich detection method in a surface antigen-specific manner with an antibody using a disk and a nano-bead, or the like can be used.

The average particle diameter of extracellular vesicles of a microalga is not particularly limited as long as the effect of the present invention is exhibited, and examples thereof include 10 nm or more, 20 nm or more, 30 nm or more, 40 nm or more, 50 nm or more, 60 nm or more, 70 nm or more, or 80 nm or more, and 700 nm or less, 600 nm or less, 500 nm or less, 450 nm or less, 400 nm or less, 350 nm or less, 300 nm or less, 250 nm or less, 200 nm or less, and 150 nm or less. Examples of the average particle diameter of extracellular vesicles of a microalga also include 10 to 700 nm, 10 to 600 nm, 10 to 500 nm, 10 to 400 nm, 10 to 300 nm, 10 to 250 nm, 30 to 700 nm, 30 to 600 nm, 30 to 500 nm, 30 to 400 nm, 30 to 300 nm, 30 to 250 nm, 50 to 700 nm, 50 to 600 nm, 50 to 500 nm, 50 to 400 nm, 50 to 300 nm, 50 to 250 nm, 70 to 700 nm, 70 to 600 nm, 70 to 500 nm, 70 to 400 nm, 70 to 300 nm, 70 to 250 nm, 100 to 700 nm, 100 to 600 nm, 100 to 500 nm, 100 to 400 nm, 100 to 300 nm, and 100 to 250 nm.

The cumulative 10% value (D10) of particle diameter among extracellular vesicles derived from Pavlova is not particularly limited as long as the effect of the present invention is exhibited, and examples thereof include 80 to 140 nm, 90 to 130 nm, and 100 to 120 nm. Examples of the cumulative 50% value (D50) of particle diameter include 120 to 210 nm, 130 to 200 nm, and 140 to 190 nm. Examples of the cumulative 90% value (D90) of particle diameter include 200 to 320 nm, 210 to 310 nm, and 220 to 300 nm. As used herein, the cumulative 10% value (D10) of particle diameter among extracellular vesicles means a diameter at which 10% of the entire extracellular vesicles are below this value. The cumulative 50% value (D50) of particle diameter among extracellular vesicles means a diameter at which 50% of the entire extracellular vesicles are below this value, and is synonymous with the median diameter. The cumulative 90% value (D90) of particle diameter among extracellular vesicles means a diameter at which 90% of the entire extracellular vesicles are below this value.

The cumulative 10% value (D10) of particle diameter among extracellular vesicles derived from Spirulina is not particularly limited as long as the effect of the present invention is exhibited, and examples thereof include 50 to 110 nm, 60 to 100 nm, and 70 to 90 nm. Examples of the cumulative 50% value (D50) of particle diameter include 100 to 160 nm, 110 to 150 nm, and 120 to 140 nm. Examples of the cumulative 90% value (D90) of particle diameter include 190 to 250 nm, 200 to 240 nm, and 210 to 230 nm.

The cumulative 10% value (D10) of particle diameter among extracellular vesicles derived from Chlorella is not particularly limited as long as the effect of the present invention is exhibited, and examples thereof include 90 to 150 nm, 100 to 140 nm, and 110 to 130 nm. Examples of the cumulative 50% value (D50) of particle diameter include 180 to 240 nm, 190 to 230 nm, and 200 to 220 nm. Examples of the cumulative 90% value (D90) of particle diameter include 320 to 380 nm, 330 to 370 nm, and 340 to 360 nm.

The content of a culture supernatant of a microalga is appropriately adjusted depending on the type and amount of other components, the dosage form, and the like, and is not limited, but can be, for example, 0.001 mass% or more with respect to the total amount of the composition, and may be 0.01 mass% or more, 0.05 mass% or more, 0.1 mass% or more, 0.5 mass% or more, 1 mass% or more, 10 mass% or more, 20 mass% or more, 30 mass% or more, 40 mass% or more, 50 mass% or more, 60 mass% or more, or 70 mass% or more. In addition, the content of a culture supernatant of a microalga can be, for example, 100 mass% or less with respect to the total amount of the composition, and examples thereof include 90 mass% or less, 80 mass% or less, 70 mass% or less, 60 mass% or less, 50 mass% or less, 40 mass% or less, 30 mass% or less, 20 mass% or less, 10 mass% or less, 1 mass% or less, and 0.1 mass% or less. Examples of the content of a culture supernatant of a microalga include 0.001 to 0.1 mass% (based on, for example, about 2 × 10¹¹ particles/mL to 4 × 10¹¹ particles/mL), 0.01 to 100 mass%, 0.01 to 90 mass%, 0.01 to 50 mass%, 0.01 to 30 mass%, 0.01 to 10 mass%, 0.01 to 1 mass%, 0.1 to 100 mass%, 0.1 to 90 mass%, 0.1 to 50 mass%, 0.1 to 30 mass%, 0.1 to 10 mass%, 0.1 to 1 mass%, 0.5 to 100 mass%, 0.5 to 90 mass%, 0.5 to 50 mass%, 0.5 to 30 mass%, 0.5 to 10 mass%, 0.5 to 1 mass%, 1 to 100 mass%, 1 to 90 mass%, 1 to 50 mass%, 1 to 30 mass%, and 1 to 10 mass% with respect to the total amount of the composition.

The content of extracellular vesicles of a microalga is appropriately adjusted depending on the type and amount of other components, the dosage form, and the like, and is not limited, but can be, for example, 0.001 mass% or more with respect to the total amount of the composition, and may be 0.005 mass% or more, 0.01 mass% or more, 0.05 mass% or more, 0.1 mass% or more, 1 mass% or more, 5 mass% or more, 10 mass% or more, or the like. In addition, the content of extracellular vesicles of a microalga can be, for example, 50 mass% or less with respect to the total amount of the composition, and examples thereof include 40 mass% or less, 30 mass% or less, 20 mass% or less, 15 mass% or less, 10 mass% or less, 1 mass% or less, and 0.1 mass% or less. Examples of the content of extracellular vesicles of a microalga include 0.001 to 0.1 mass% (based on, for example, about 2 × 10¹¹ particles/mL to 4 × 10¹¹ particles/mL), 0.001 to 50 mass%, 0.001 to 40 mass%, 0.001 to 30 mass%, 0.001 to 20 mass%, 0.001 to 15 mass%, 0.01 to 50 mass%, 0.01 to 40 mass%, 0.01 to 30 mass%, 0.01 to 20 mass%, 0.01 to 15 mass%, 0.1 to 50 mass%, 0.1 to 40 mass%, 0.1 to 30 mass%, 0.1 to 20 mass%, 0.1 to 15 mass%, 1 to 50 mass%, 1 to 40 mass%, 1 to 30 mass%, 1 to 20 mass%, 1 to 15 mass%, 5 to 50 mass%, 5 to 40 mass%, 5 to 30 mass%, 5 to 20 mass%, and 5 to 15 mass% with respect to the total amount of the composition.

The content of extracellular vesicles of a microalga is appropriately adjusted depending on the type and amount of other components, the dosage form, and the like, and is not limited, but can be, for example, as the protein amount, 0.01 µg/mL or more, 0.1 µg/mL or more, 1 µg/mL or more, 10 µg/mL or more, or 100 µg/mL or more with respect to the total amount of the composition. In addition, the content includes 100,000 µg/mL or less, 10,000 µg/mL or less, 1000 µg/mL, 100 µg/mL or less, or 10 µg/mL or less.

### [Application]

In one embodiment, the composition of the present invention is suitably used for maintaining or increasing fibrous structural proteins. In the present specification, the fibrous structural proteins refer to a fibrous component in the body and a component involved in the tissue structure thereof. Without limitation, the fibrous structural proteins are preferably proteins related to skin, more preferably proteins related to epidermis or dermis, still more preferably proteins related to dermis. In another embodiment, the fibrous structural proteins are preferably proteins related to elasticity of skin.

As described above, skin is roughly divided into epidermis and dermis, and the constituent components of dermis are classified into a stromal component related to the fibrous tissue and a cellular component. As the stromal component related to the fibrous tissue, collagen fibers (herein, also simply referred to as collagen) such as collagen type I and type III account for the most part, and further, elastic fibers composed of elastin, fibrillin and the like, and substrates such as hyaluronic acid are included. These are collectively called extracellular matrix (ECM).

Examples of the fibrous structural proteins include proteoglycans such as collagen, elastin, and hyaluronic acid, glycosaminoglycans, laminin, and fibronectin.

There are about 28 subtypes of collagen, but about 80% of dermis-constituting collagen is known to be type I. Collagen type I contributes to elasticity of skin and the like, and collagen type III contributes to recovery from skin damage and the like. Collagen is important as a supporting tissue for maintaining the strength of skin because collagen has a strong resistance force against the tension acting particularly along the running of fibers and is poor in extensibility. Elastic fibers are composed of elastin and fibrillin, and are involved in elasticity of skin and extensibility of arteries and tendons by being interposed between layer structures created by collagen. Hyaluronic acid is a type of proteoglycan, is present between fibrous components such as collagen and elastin and cells, and is involved in stabilizing the fibrous structure and retaining moisture. Glycoproteins such as laminin and fibronectin mediate adhesion between ECM and cells.

Activation of a fibrous structural protein decomposition enzyme may reduce the fibrous structural proteins described above. Examples of the fibrous structural protein decomposition enzyme include matrix metalloprotease (MMPS), and in particular, matrix metalloprotease-1 (MMP-1) is a cause of degrading collagen and elastin fibers of skin.

In addition, when the activity of a fibrous structural protein synthase or the gene expression of the fibrous structural protein is reduced, the fibrous structural proteins may be reduced. Examples of the fibrous structural protein synthase include a hyaluronic acid synthase. As the hyaluronic acid synthase (herein, also referred to as HAS), HAS-1 to 3 are known, and hyaluronic acid is directly synthesized from UDP (uridine diphosphate)-glucuronic acid (UDP-GlcA) and UDP-N-acetyl-D-glucosamine (UDP-GlcNAc) by these enzymes on a cell membrane and supplied to the outside of the cells. In epidermis, HAS-3 mainly contributes, but in dermis, HAS-1 and HAS-2 contribute, especially HAS-2 greatly contributes.

Examples of genes of the fibrous structural proteins include, in particular, in skin collagen, collagen type I-related genes and collagen type III-related genes, including COL1A1 (collagen type I alpha 1 chain) and COL3A1 (collagen type III alpha 1 chain).

Accordingly, a component capable of suppressing a fibrous structural protein decomposition enzyme, activating a fibrous structural protein synthase, or enhancing the gene expression of the fibrous structural protein leads to maintaining the fibrous structural protein by suppressing a reduction in fibrous structural protein, or increasing fibrous structural protein.

In Examples described later, it has been confirmed that a culture supernatant and/or extracellular vesicles of a microalga suppress matrix metalloprotease-1 (MMP-1), enhance the gene expression of elastin, enhance the gene expression of COL1A1, enhance the gene expression of COL3A1, activate hyaluronic acid synthase HAS-1, or activate hyaluronic acid synthase HAS-2. These results are expected to contribute to maintenance or increase of the fibrous structural proteins, leading to improvement of anti-aging of skin, specifically, skin elasticity, firmness, wrinkles, skin texture, moisture, and the like, and leading to suppression of dryness, roughness, and the like of skin.

The composition of the present invention can be added to or mixed with a pharmaceutical product, a quasi-pharmaceutical product, a cosmetic product, a food product, a beverage, a feed, or a pet food. Alternatively, the composition of the present invention can be used as it is as a pharmaceutical product, a quasi-pharmaceutical product, a cosmetic product, a food product, a beverage, a feed, a pet food, or the like.

When the composition of the present invention is added to or mixed with a so-called external preparation such as a pharmaceutical product, a quasi-pharmaceutical product, or a cosmetic product, the composition of the present invention can be used as a pharmaceutical product, a quasi-pharmaceutical product, or a cosmetic product by which improvement of skin elasticity, skin firmness, wrinkles, skin texture, moisture, dryness, roughness, or the like is clearly indicated or implied as an efficacy, an effect or function.

When the above-mentioned functionality is not clearly indicated, it is also possible to use the composition of the present invention with indications directed to, for example, those seeking elasticity of the skin, those seeking firmness of the skin, those concerned about wrinkles, those concerned about fine lines, those wishing to refine skin texture, those seeking moisture of the skin, those concerned about dryness of the skin, those concerned about roughness of the skin, those concerned about pores of the skin, those concerned about an unstable skin condition, those concerned about pores of the skin, or with similar indications.

When used in a so-called external preparation such as a pharmaceutical product, a quasi-pharmaceutical product, or a cosmetic product, the adult daily dose of extracellular vesicles of a microalga can be appropriately determined depending on the condition of the individual, body weight, sex, age, activity of the material, ingestion or route of administration, ingestion or administration schedule, formulation form, or other factors. Examples of the adult daily dose of extracellular vesicles of a microalga include 1 µg or more, 5 µg or more, 10 µg or more, 15 µg or more, 20 µg or more, 30 µg or more, 40 µg or more, 50 µg or more, 70 µg or more, 100 µg or more, 150 µg or more, and 200 µg or more. Examples of the adult daily dose of extracellular vesicles of microalgae include 5 mg or less, 3 mg or less, 1 mg or less, 900 µg or less, 800 µg or less, 700 µg or less, 600 µg or less, 500 µg or less, 400 µg or less, 300 µg or less, 200 µg or less, and 100 µg or less. In addition, examples of the adult daily dose of extracellular vesicles of a microalga include 1 to 1000 µg, 1 to 900 µg, 1 to 800 µg, 1 to 700 µg, 1 to 600 µg, 1 to 500 µg, 1 to 400 µg, 1 to 300 µg, 1 to 200 µg, 1 to 100 µg, 5 to 1000 µg, 5 to 900 µg, 5 to 800 µg, 5 to 700 µg, 5 to 600 µg, 5 to 500 µg, 5 to 400 µg, 5 to 300 µg, 5 to 200 µg, 5 to 100 µg, 10 to 1000 µg, 10 to 900 µg, 10 to 800 µg, 10 to 700 µg, 10 to 600 µg, 10 to 500 µg, 10 to 400 µg, 10 to 300 µg, 10 to 200 µg, 10 to 100 µg, 20 to 1000 µg, 20 to 900 µg, 20 to 800 µg, 20 to 700 µg, 20 to 600 µg, 20 to 500 µg, 20 to 400 µg, 20 to 300 µg, 20 to 200 µg, and 20 to 100 µg. In addition, the dose of extracellular vesicles of a microalga varies depending on various factors such as the condition and age of the subject, but when an adult is used as a reference, generally, 1 µg/kg to 200 mg/kg of the composition, or 50 µg/kg to 50 mg/kg in another aspect may be administered 1 to 3 times a day, and the dose does not limit the range of the present specification by any method.

When used in a so-called external preparation such as a pharmaceutical product, a quasi-pharmaceutical product, or a cosmetic product, the adult daily dose of extracellular vesicles of a microalga can be appropriately determined depending on the condition of the individual, body weight, sex, age, activity of the material, ingestion or route of administration, ingestion or administration schedule, formulation form, or other factors. Examples of the adult daily dose of extracellular vesicles of a microalga include 1 µg or more, 5 µg or more, 10 µg or more, 15 µg or more, 20 µg or more, 30 µg or more, 40 µg or more, 50 µg or more, 70 µg or more, 100 µg or more, 150 µg or more, and 200 µg or more as the protein amount. Examples of the adult daily dose of extracellular vesicles of microalgae include 5 mg or less, 3 mg or less, 1 mg or less, 900 µg or less, 800 µg or less, 700 µg or less, 600 µg or less, 500 µg or less, 400 µg or less, 300 µg or less, 200 µg or less, and 100 µg or less. In addition, examples of the adult daily dose of extracellular vesicles of a microalga include 1 to 1000 µg, 1 to 900 µg, 1 to 800 µg, 1 to 700 µg, 1 to 600 µg, 1 to 500 µg, 1 to 400 µg, 1 to 300 µg, 1 to 200 µg, 1 to 100 µg, 5 to 1000 µg, 5 to 900 µg, 5 to 800 µg, 5 to 700 µg, 5 to 600 µg, 5 to 500 µg, 5 to 400 µg, 5 to 300 µg, 5 to 200 µg, 5 to 100 µg, 10 to 1000 µg, 10 to 900 µg, 10 to 800 µg, 10 to 700 µg, 10 to 600 µg, 10 to 500 µg, 10 to 400 µg, 10 to 300 µg, 10 to 200 µg, 10 to 100 µg, 20 to 1000 µg, 20 to 900 µg, 20 to 800 µg, 20 to 700 µg, 20 to 600 µg, 20 to 500 µg, 20 to 400 µg, 20 to 300 µg, 20 to 200 µg, and 20 to 100 µg. In addition, the dose of extracellular vesicles of a microalga varies depending on various factors such as the condition and age of the subject, but when an adult is used as a reference, 1 µg/kg to 200 mg/kg of the composition, or 50 µg/kg to 50 mg/kg in another aspect may be administered 1 to 3 times a day, and the dose does not limit the range of the present specification by any method.

In another embodiment, the adult daily dose of extracellular vesicles of a microalga is, for example, 1 × 10⁷ or more, 1 × 10⁸ or more, or 1 × 10⁹ or more in terms of the number of vesicles. In addition, the adult daily dose of extracellular vesicles of a microalga is, for example, 1 × 10¹⁴ or less, 1 × 10¹³ or less, 1 × 10¹² or less, 1 × 10¹¹ or less, or 1 × 10¹⁰ or less in terms of the number of vesicles. Examples of the adult daily dose of extracellular vesicles of a microalga include 1 × 10⁷ to 1 × 10¹⁴, 1 × 10⁷ to 1 × 10¹³, 1 × 10⁷ to 1 × 10¹², 1 × 10⁷ to 1 × 10¹¹, 1 × 10⁸ to 1 × 10¹⁴, 1 × 10⁸ to 1 × 10¹³, 1 × 10⁸ to 1 × 10¹², 1 × 10⁸ to 1 × 10¹¹, 1 × 10⁹ to 1 × 10¹⁴, 1 × 10⁹ to 1 × 10¹³, 1 × 10⁹ to 1 × 10¹², and 1 × 10⁹ to 1 × 10¹¹ in terms of the number of vesicles.

The adult daily dose of the culture supernatant of a microalga can be appropriately determined according to the condition of the individual, body weight, sex, age, activity of the material, administration schedule, formulation form, or other factors. The adult daily dose of the culture supernatant of a microalga can be, for example, an amount in which extracellular vesicles of a microalga are contained in the above-described intake amount or dose.

The subject targeted by the composition of the present invention is not particularly limited as long as the subject is a subject in need of maintaining or increasing the fibrous structural proteins, but may be, for example, young people in their 20's to 30's.

The composition of the present invention may be taken or administered in 1 to several portions per day, usually 1 to 6 times per day, 1 to 3 times per day, 1 to 2 times per day, or at any period and interval, but preferably once per day.

The composition of the present invention can be used as a food or beverage product clearly or implicitly indicating improvement of skin elasticity, skin firmness, wrinkles, skin texture, moisture, dryness, roughness, or the like as functionality, that is, a health food product, a food product labeled with functionality, a food product for patients, and a food product for specified health uses. In addition, the composition of the present invention can be used as a so-called doctor's supplement recommended or presented by a doctor in a hospital and/or a doctor's office, or internal medicine, orthopedic surgery, animal hospital, or the like even when the functionality is not clearly indicated or implied.

When the above-mentioned functionality is not clearly indicated, it is also possible to use the composition of the present invention with indications directed to, for example, those seeking elasticity of the skin, those seeking firmness of the skin, those concerned about wrinkles, those concerned about fine lines, those wishing to refine skin texture, those seeking moisture of the skin, those concerned about dryness of the skin, those concerned about roughness of the skin, those concerned about pores of the skin, those concerned about an unstable skin condition, those concerned about pores of the skin, or with similar indications.

The composition of the present invention can be applied in the form of oral use, internal use, and the like. When used as a pharmaceutical composition, the composition of the present invention may be used therapeutically or non-therapeutically.

The adult daily oral intake amount or dose of extracellular vesicles of a microalga can be appropriately determined depending on the condition of the individual, body weight, sex, age, activity of the material, ingestion or route of administration, ingestion or administration schedule, formulation form, or other factors. Examples of the adult daily oral intake amount or dose of extracellular vesicles of a microalga include 1 µg or more, 5 µg or more, 10 µg or more, 15 µg or more, 20 µg or more, 30 µg or more, 40 µg or more, 50 µg or more, 70 µg or more, 100 µg or more, 150 µg or more, and 200 µg or more. Examples of the adult daily oral intake amount or dose of extracellular vesicles of a microalga include 5 mg or less, 3 mg or less, 1 mg or less, 900 µg or less, 800 µg or less, 700 µg or less, 600 µg or less, 500 µg or less, 400 µg or less, 300 µg or less, 200 µg or less, and 100 µg or less. In addition, examples of the adult daily oral intake amount or dose of extracellular vesicles of a microalga include 1 to 1000 µg, 1 to 900 µg, 1 to 800 µg, 1 to 700 µg, 1 to 600 µg, 1 to 500 µg, 1 to 400 µg, 1 to 300 µg, 1 to 200 µg, 1 to 100 µg, 5 to 1000 µg, 5 to 900 µg, 5 to 800 µg, 5 to 700 µg, 5 to 600 µg, 5 to 500 µg, 5 to 400 µg, 5 to 300 µg, 5 to 200 µg, 5 to 100 µg, 10 to 1000 µg, 10 to 900 µg, 10 to 800 µg, 10 to 700 µg, 10 to 600 µg, 10 to 500 µg, 10 to 400 µg, 10 to 300 µg, 10 to 200 µg, 10 to 100 µg, 20 to 1000 µg, 20 to 900 µg, 20 to 800 µg, 20 to 700 µg, 20 to 600 µg, 20 to 500 µg, 20 to 400 µg, 20 to 300 µg, 20 to 200 µg, and 20 to 100 µg.

In another embodiment, the adult daily oral intake amount or dose of extracellular vesicles of a microalga is, for example, 1 × 10⁷ or more, 1 × 10⁸ or more, or 1 × 10⁹ or more in terms of the number of vesicles. In addition, the adult daily oral intake amount or dose of extracellular vesicles of a microalga is, for example, 1 × 10¹⁴ or less, 1 × 10¹³ or less, 1 × 10¹² or less, 1 × 10¹¹ or less, or 1 × 10¹⁰ or less in terms of the number of vesicles. Examples of the adult daily oral intake amount or dose of extracellular vesicles of a microalga include 1 × 10⁷ to 1 × 10¹⁴, 1 × 10⁷ to 1 × 10¹³, 1 × 10⁷ to 1 × 10¹², 1 × 10⁷ to 1 × 10¹¹, 1 × 10⁸ to 1 × 10¹⁴, 1 × 10⁸ to 1 × 10¹³, 1 × 10⁸ to 1 × 10¹², 1 × 10⁸ to 1 × 10¹¹, 1 × 10⁹ to 1 × 10¹⁴, 1 × 10⁹ to 1 × 10¹³, 1 × 10⁹ to 1 × 10¹², and 1 × 10⁹ to 1 × 10¹¹ in terms of the number of vesicles.

The adult daily oral intake amount or dose of the culture supernatant of a microalga can be appropriately determined depending on the condition of the individual, body weight, sex, age, activity of the material, ingestion or route of administration, ingestion or administration schedule, formulation form, or other factors. The adult daily oral intake amount or dose of the culture supernatant of a microalga can be, for example, an amount in which extracellular vesicles of a microalga are contained in the above-described intake amount or dose.

The adult daily oral intake amount or dose may be divided depending on the dosage form, for example, into 1 to 6 capsules, 1 to 4 capsules, 1 to 3 capsules, or 1 to 2 capsules, in the case of capsules.

The composition of the present invention may be taken or administered in 1 to several portions per day, usually 1 to 6 times per day, 1 to 3 times per day, 1 to 2 times per day, or at any period and interval, but preferably once per day.

When the present invention is used as a feed, a pet food, or the like, the target organism is not particularly limited, but is preferably mammal, reptile, amphibian, bird, or fish, more preferably mammal other than human. For example, the target organism includes platypus, spiny anteater, opossum, native cat, kangaroo, aardvark, hyrax, elephant, armadillo, sloth, anteater, tree shrew, flying lemur, chimpanzee, rabbit, degu, dormouse, squirrel, raccoon, mouse, hedgehog, chinchilla, ferret, camel, wild boar, giraffe, deer, cattle, goat, hippopotamus, whale, dolphin, horse, rhinoceros, tapir, bat, monkey, tiger, wolf, weasel, bear, seal, dog, cat, parakeet, parrot, finch, owl, and eared owl. Among them, dog or cat is more preferable.

When the present invention is used as a feed, a pet food, or the like, it may be fed several times a day by adding it to a staple food or the like, or may be given as a snack as needed.

The composition of the present invention includes the following aspects.
[1] A composition for maintaining or increasing a fibrous structural protein, containing a culture supernatant and/or extracellular vesicles of a microalga.
[2] A composition for inhibiting a fibrous structural protein decomposition enzyme, containing a culture supernatant and/or extracellular vesicles of a microalga.
[3] A composition for activating a fibrous structural protein synthase, containing a culture supernatant and/or extracellular vesicles of a microalga.
[4] A composition for enhancing gene expression of a fibrous structural protein, containing a culture supernatant and/or extracellular vesicles of a microalga.
[5] The composition according to any of [1] to [4], wherein the fibrous structural protein is collagen, elastin, or hyaluronic acid.
[6] The composition according to [5], wherein the elastin is skin elastin.
[7] A composition for maintaining or increasing skin elasticity, containing a culture supernatant and/or extracellular vesicles of a microalga.
[8] A composition for promoting production of collagen, elastin, or hyaluronic acid, containing a culture supernatant and/or extracellular vesicles of a microalga.
[9] A composition for inhibiting matrix metalloprotease-1 (MMP-1), containing a culture supernatant and/or extracellular vesicles of a microalga.
[10] A composition for enhancing gene expression of elastin, containing a culture supernatant and/or extracellular vesicles of a microalga.
[11] A composition for enhancing gene expression of COL1A1, containing a culture supernatant and/or extracellular vesicles of a microalga.
[12] A composition for enhancing gene expression of COL3A1, containing a culture supernatant and/or extracellular vesicles of a microalga.
[13] A composition for activating hyaluronic acid synthase HAS-1, containing a culture supernatant and/or extracellular vesicles of a microalga.
[14] A composition for activating hyaluronic acid synthase HAS-2, containing a culture supernatant and/or extracellular vesicles of a microalga.
[15] The composition according to any of [1] to [14], wherein the culture supernatant of a microalga is obtained by a method including purification through filtration with a filter having a membrane pore diameter of 0.001 µm to 0.2 µm.
[16] The composition according to any of [1] to [15], wherein the filter has a molecular weight cutoff of 10 KDa to 1000 KDa.
[17] The composition according to any of [1] to [16], wherein particle diameter of the extracellular vesicles is 50 nm to 700 nm.
[18] The composition according to any of [1] to [17], wherein a unit formulation of the composition contains 0.001 to 0.1 mass% of the culture supernatant and/or extracellular vesicles of a microalga derived from the microalga.
[19] The composition according to any of [1] to [18], wherein a unit formulation of the composition contains 1.0×10⁶ to 1.0×10¹¹ extracellular vesicles/mL derived from the microalga.
[20] The composition according to any of [1] to [19], wherein the microalga is a microalga belonging to the genus Pavlova, Euglena, Spirulina, or Chlorella.
[21] The composition according to any of [1] to [20], wherein the culture supernatant and/or extracellular vesicles of a microalga derived from the microalga are provided as a food or beverage, a cosmetic, a pharmaceutical product, or a quasi-pharmaceutical product.
[22] The composition according to any of [1] to [21], wherein the culture supernatant and/or extracellular vesicles of a microalga derived from the microalga are provided for young people.

The present invention further includes the following aspects. More specific embodiments of each aspect are the same as those described in the section of [Composition for maintaining or increasing fibrous structural protein] above.
[1] Use of a culture supernatant and/or extracellular vesicles of a microalga for production of a composition for maintaining or increasing a fibrous structural protein.
[2] Use of a culture supernatant and/or extracellular vesicles of a microalga for production of a composition for inhibiting a fibrous structural protein decomposition enzyme.
[3] Use of a culture supernatant and/or extracellular vesicles of a microalga for production of a composition for activating a fibrous structural protein synthase.
[4] Use of a culture supernatant and/or extracellular vesicles of a microalga for production of a composition for enhancing gene expression of a fibrous structural protein.
[5] The use according to any of [1] to [4], wherein the fibrous structural protein is collagen, elastin, or hyaluronic acid.
[6] The use according to [5], wherein the elastin is skin elastin.
[7] Use of a culture supernatant and/or extracellular vesicles of a microalga for production of a composition for maintaining or increasing skin elasticity.
[8] Use of a culture supernatant and/or extracellular vesicles of a microalga for production of a composition for promoting production of collagen, elastin, or hyaluronic acid.
[9] Use of a culture supernatant and/or extracellular vesicles of a microalga for production of a composition for inhibiting matrix metalloprotease-1 (MMP-1).
[10] Use of a culture supernatant and/or extracellular vesicles of a microalga for production of a composition for enhancing gene expression of elastin.
[11] Use of a culture supernatant and/or extracellular vesicles of a microalga for production of a composition for enhancing gene expression of COL1A1.
[12] Use of a culture supernatant and/or extracellular vesicles of a microalga for production of a composition for enhancing gene expression of COL3A1.
[13] Use of a culture supernatant and/or extracellular vesicles of a microalga for production of a composition for activating hyaluronic acid synthase HAS-1.
[14] Use of a culture supernatant and/or extracellular vesicles of a microalga for production of a composition for activating hyaluronic acid synthase HAS-2.
[15] The use according to any of [1] to [14], wherein the culture supernatant of a microalga is obtained by a method including purification through filtration with a filter having a membrane pore diameter of 0.001 µm to 0.2 µm.
[16] The use according to any of [1] to [15], wherein the filter has a molecular weight cutoff of 10 KDa to 1000 KDa.
[17] The use according to any of [1] to [16], wherein particle diameter of the extracellular vesicles is 50 nm to 700 nm.
[18] The use according to any of [1] to [17], wherein a unit formulation of the composition contains 0.001 to 0.1 mass% of the culture supernatant and/or extracellular vesicles of a microalga derived from the microalga.
[19] The use according to any of [1] to [18], wherein a unit formulation of the composition contains 1.0×10⁶ to 1.0×10¹¹ extracellular vesicles/mL derived from the microalga.
[20] The use according to any of [1] to [19], wherein the microalga is a microalga belonging to the genus Pavlova, Euglena, Spirulina, or Chlorella.
[21] The use according to any of [1] to [20], wherein the culture supernatant and/or extracellular vesicles of a microalga derived from the microalga are provided as a food or beverage, a cosmetic, a pharmaceutical product, or a quasi-pharmaceutical product.
[22] The use according to any of [1] to [21], wherein the culture supernatant and/or extracellular vesicles of a microalga derived from the microalga are provided for young people.
[23] A method for maintaining or increasing a fibrous structural protein, including administering to or allowing a subject to ingest an effective amount of a culture supernatant and/or extracellular vesicles of a microalga.
[24] A method for inhibiting a fibrous structural protein decomposition enzyme, including administering to or allowing a subject to ingest an effective amount of a culture supernatant and/or extracellular vesicles of a microalga.
[25] A method for activating a fibrous structural protein synthase, including administering to or allowing a subject to ingest an effective amount of a culture supernatant and/or extracellular vesicles of a microalga.
[26] A method for enhancing gene expression of a fibrous structural protein, including administering to or allowing a subject to ingest an effective amount of a culture supernatant and/or extracellular vesicles of a microalga.
[27] The method according to any of [23] to [26], wherein the fibrous structural protein is collagen, elastin, or hyaluronic acid.
[28] The method according to [27], wherein the elastin is skin elastin.
[29] A method for maintaining or increasing skin elasticity, including administering to or allowing a subject to ingest an effective amount of a culture supernatant and/or extracellular vesicles of a microalga.
[30] A method for promoting production of collagen, elastin, or hyaluronic acid, including administering to or allowing a subject to ingest an effective amount of a culture supernatant and/or extracellular vesicles of a microalga.
[31] A method for inhibiting matrix metalloprotease-1 (MMP-1), including administering to or allowing a subject to ingest an effective amount of a culture supernatant and/or extracellular vesicles of a microalga.
[32] A method for enhancing gene expression of elastin, including administering to or allowing a subject to ingest an effective amount of a culture supernatant and/or extracellular vesicles of a microalga.
[33] A method for enhancing gene expression of COL1A1, including administering to or allowing a subject to ingest an effective amount of a culture supernatant and/or extracellular vesicles of a microalga.
[34] A method for enhancing gene expression of COL3A1, including administering to or allowing a subject to ingest an effective amount of a culture supernatant and/or extracellular vesicles of a microalga.
[35] A method for activating hyaluronic acid synthase HAS-1, including administering to or allowing a subject to ingest an effective amount of a culture supernatant and/or extracellular vesicles of a microalga.
[36] A method for activating hyaluronic acid synthase HAS-2, including administering to or allowing a subject to ingest an effective amount of a culture supernatant and/or extracellular vesicles of a microalga.
[37] The method according to any of [23] to [36], wherein the culture supernatant of a microalga is obtained by a method including purification through filtration with a filter having a membrane pore diameter of 0.001 µm to 0.2 µm.
[38] The method according to any of [23] to [37], wherein the filter has a molecular weight cutoff of 10 KDa to 1000 KDa.
[39] The method according to any of [23] to [38], wherein particle diameter of the extracellular vesicles is 50 nm to 700 nm.
[40] The method according to any of [23] to [39], wherein a unit formulation of the composition contains 0.001 to 0.1 mass% of the culture supernatant and/or extracellular vesicles of a microalga derived from the microalga.
[41] The method according to any of [23] to [40], wherein a unit formulation of the composition contains a content of 1.0×10⁶ to 1.0×10¹¹ extracellular vesicles/mL derived from the microalga.
[42] The method according to any of [23] to [41], wherein the microalga is a microalga belonging to the genus Pavlova, Euglena, Spirulina, or Chlorella.
[43] The method according to any of [23] to [42], wherein the culture supernatant and/or extracellular vesicles of a microalga derived from the microalga are provided as a food or beverage, a cosmetic, a pharmaceutical product, or a quasi-pharmaceutical product.
[44] The method according to any of [23] to [43], wherein the culture supernatant and/or extracellular vesicles of a microalga derived from the microalga are provided for young people.

### EXAMPLES

Next, a specific description is made of the present invention with reference to Examples, but the present invention is not limited to the following Examples.

### [Test Example 1. Preparation and evaluation of culture supernatant and/or extracellular vesicles of microalga]

Using a commercially available medium for algae, Pavlova sp., Arthrospira platensis, or Chlorella vulgaris was cultured by a conventional method to yield a culture supernatant.

Using 500 mL of each culture supernatant, concentration was performed to a volume of 5 mL or less with a 200 K ultrafiltration membrane (manufactured by ADVANTEC). Thereafter, concentrating washing was performed 3 times using 100 mL of PBS, and collection was finally conducted with 50 mL of PBS. Thereafter, each concentrated sample was subjected to filter sterilization using a 0.22 µm filter, and the number of particles and the like was measured using NanoSight.

Each of the samples was appropriately diluted with PBS(-), and a number distribution graph (horizontal axis: particle diameter, vertical axis: particle number concentration) was created according to the attached software using a nanoparticle analysis system NanoSight LM10 (manufactured by Malvern Panalytical).

The following samples were obtained as described above. Based on these samples, the samples were diluted 10-fold, 100-fold, 1000-fold, or 10,000-fold for addition to cells in the following Test Examples.
- Pavlova sp. culture supernatant ultrafiltrated sample (protein concentration: 11.9 µg/mL, also denoted as "PavloEV" in the figure)
- Arthrospira platensis culture supernatant ultrafiltrated sample (protein concentration: 29.6 µg/mL, also denoted as "SpirulEV" in the figure)
- Chlorella vulgaris culture supernatant ultrafiltrated sample (protein concentration: 10.4 µg/mL, also denoted as "ChloreEV" in the figure)

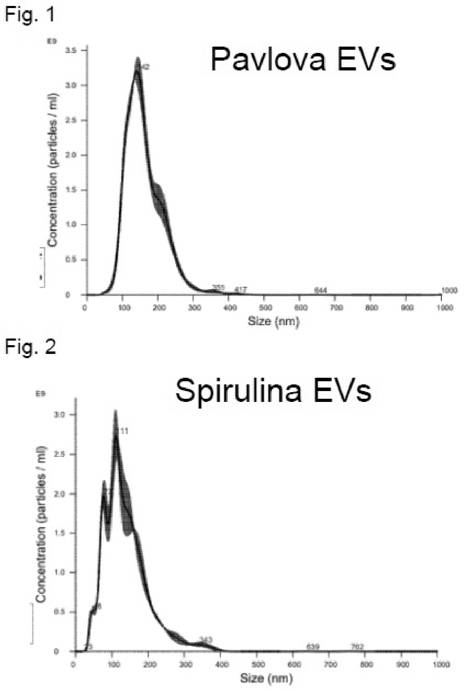

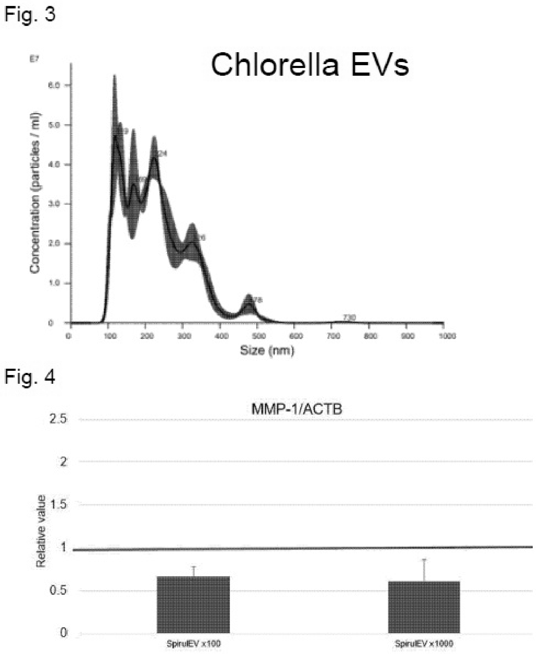

The result of the Pavlova sp. culture supernatant ultrafiltrated sample is shown in Fig. 1, the result of the Arthrospira platensis culture supernatant ultrafiltrated sample is shown in Fig. 2, and the result of the Chlorella vulgaris culture supernatant ultrafiltrated sample is shown in Fig. 3, respectively.

As shown in Fig. 1, in the Pavlova sp. culture supernatant ultrafiltrated sample, the number of particles of Pavlova sp.-derived extracellular vesicles was 3.32 × 10¹¹/mL, the average particle diameter was 162.8 nm, the cumulative 10% value of particle diameter (D10) was 105.4 nm, the cumulative 50% value of particle diameter (D50) was 151.9 nm, and the cumulative 90% value of particle diameter (D90) was 232.3 nm.

As shown in Fig. 2, in the Arthrospira platensis culture supernatant ultrafiltrated sample, the number of particles of Arthrospira platensis-derived extracellular vesicles was 2.88 × 10¹¹/mL, the average particle diameter was 138.7 nm, the cumulative 10% value of particle diameter (D10) was 72.2 nm, the cumulative 50% value of particle diameter (D50) was 125.9 nm, and the cumulative 90% value of particle diameter (D90) was 217.8 nm.

As shown in Fig. 3, in the Chlorella vulgaris culture supernatant ultrafiltrated sample, the number of particles of Chlorella vulgaris-derived extracellular vesicles was 8.18 × 10⁹/mL, the average particle diameter was 227.7 nm, the cumulative 10% value of particle diameter (D10) was 121.4 nm, the cumulative 50% value of particle diameter (D50) was 215.8 nm, and the cumulative 90% value of particle diameter (D90) was 348.5 nm.

### [Test example 2: Test for adding culture supernatant and/or extracellular vesicles of microalga to human fibroblasts]

Each of the ultrafiltrated samples obtained in Test Example 1 was diluted 10-fold, 100-fold, 1000-fold, or 10,000-fold to be allowed to act on human fibroblasts (CC-2509, manufactured by LONZA). Thereafter, culturing was performed in a 5%CO₂ incubator at 37°C for 48 hours.

The human fibroblasts after 48 hours of treatment of each sample, from which the medium was removed, were washed once. QIAzol (manufactured by QIAGEN) was added at 700 mL/well. The mixture was collected in a 1.5 mL tube with a cell scraper and stored at -80°C. Thereafter, RNA was extracted with miRNeasy mini Kit (manufactured by QIAGEN).

The RNA sample was subjected to DNase treatment after measurement of the RNA concentration with NanoDrop ND-1000 (manufactured by Thermo Fisher Scientific), and then cDNAized through reverse transcription reaction.

The cDNA sample after completion of the reverse transcription was diluted 5-fold with RNase free water and then stored at -30°C.

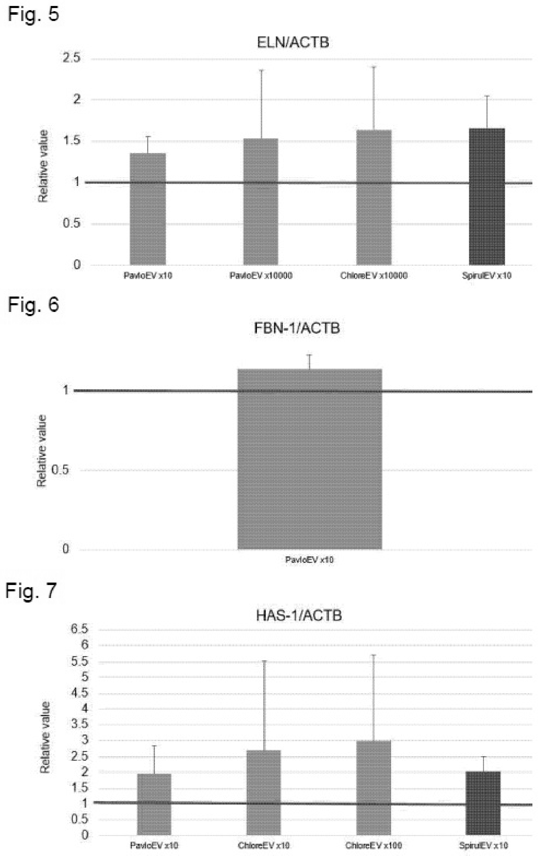

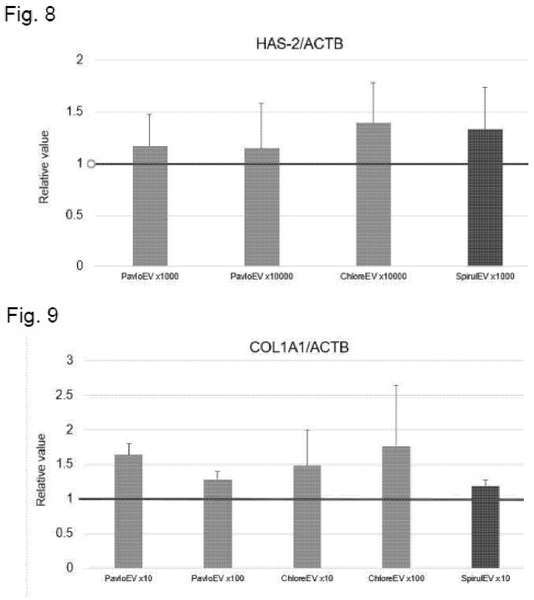

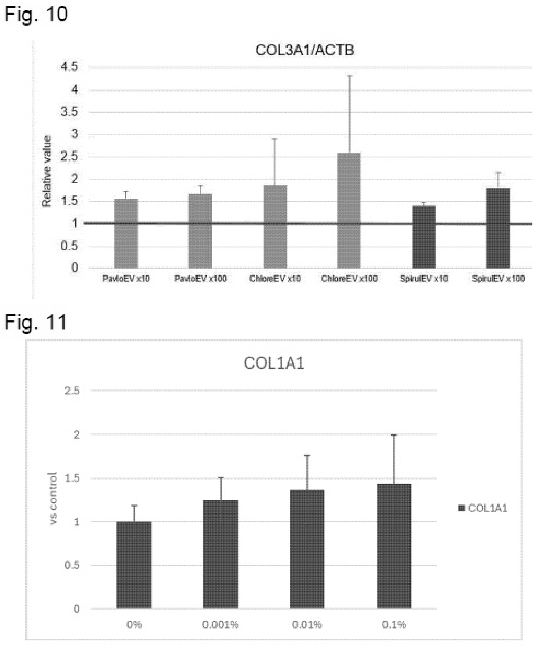

Gene expression analysis of MMP1, elastin (also denoted as ELN), fibrillin 1 (also denoted as FBN-1), hyaluronic acid synthase 1 (also denoted as HAS-1), hyaluronic acid synthase 2 (also denoted as HAS-2), COL1A1 (collagen type I alpha 1 chain), and COL3A1 (collagen type III alpha 1 chain) was performed by a Real-Time PCR method using TaqMan probe by QuantStudio (registered trademark) 3 real-time PCR system (manufactured by Thermo Fisher Scientific). A case where each sample was not added was used as a control (a sample to which only PBS was added), and β-actin (also denoted as ACTB) was used as an internal standard. Data analysis was performed by using a ΔΔCT method. The gene expression result of MMP1 is shown in Fig. 4, the gene expression result of elastin is shown in Fig. 5, the gene expression result of fibrillin 1 is shown in Fig. 6, the gene expression result of hyaluronic acid synthase 1 is shown in Fig. 7, the gene expression result of hyaluronic acid synthase 2 is shown in Fig. 8, the gene expression result of COL1A1 is shown in Fig. 9, and the gene expression result of COL3A1 is shown in Fig. 10.

As shown in Figs. 4 to 10, it has been confirmed that allowing the culture supernatant and/or extracellular vesicles of a microalga to act on human fibroblasts suppresses matrix metalloprotease-1 (MMP-1), enhances the gene expression of elastin, enhances the gene expression of COL1A1, enhances the gene expression of COL3A1, activates hyaluronic acid synthase HAS-1, or activates hyaluronic acid synthase HAS-2 in the human fibroblasts. Accordingly, the culture supernatant and/or extracellular vesicles of a microalga can be suitably used for maintaining or increasing fibrous structural proteins, and can be suitably used for improving anti-aging of the skin, specifically skin elasticity, skin firmness, wrinkles, skin texture, moisture, and the like.

### [Test example 3: Test for adding culture supernatant and/or extracellular vesicles of microalga to three-dimensional cultured skin]

### (Pre-culture of three-dimensional cultured skin)

In this test example, evaluation was performed using three-dimensional cultured skin (EFT-400, manufactured by KURABO INDUSTRIES LTD.). To the sterilized 6 well receiver plate (hereinafter, the plate) in the EFT-400 kit, 2.5 ml of a medium (EFT-400 ASY or EFT-400-MM, manufactured by KURABO INDUSTRIES LTD.) dedicated to EFT-400 at 37°C was dispensed into the wells, and the surface of each well was confirmed to be uniformly covered with the medium.

Next, the three-dimensional cultured skin was transferred from the culture cup stored at 4°C to the plate to which the medium had been added. Thereafter, the skin was left to stand and cultured in a 5%CO₂ incubator at 37°C for about 16 to 18 hours.

### (Preparation of composition containing extracellular vesicles derived from Pavlova sp.)

Pavlova sp. was cultured by a conventional method using a commercially available medium for algae to yield a culture supernatant. The culture supernatant was heat-treated at 105°C for 4 minutes. After heating, 3000 mL of the supernatant obtained through centrifugation at room temperature was filtered through a 0.2 µm filter, and the filtrate was concentrated to a volume of 150 mL or less by a tangential flow method using a 300 kDa hollow fiber membrane filter (MiniKros sampler S02-E300-05-N, manufactured by Repligen). Thereafter, concentrating washing was performed using 10,000 mL of PBS, and collection was finally conducted using 100 mL of PBS. Thereafter, the concentrated sample was subjected to filter sterilization using a 0.22 µm filter to yield a composition containing the extracellular vesicles (ultrafiltrated sample) derived from Pavlova sp.

Extracellular vesicles derived from Pavlova sp. in ultrafiltrated sample: 2.7 × 10¹² extracellular vesicles/mL, average particle diameter 158.8 nm, protein concentration 836 µg/ml

### (Addition of ultrafiltrated sample)

The three-dimensional cultured skin was pre-cultured, and then the medium was replaced once. Thereafter, the composition containing the extracellular vesicles (ultrafiltrated sample) derived from Pavlova sp. was added to the three-dimensional cultured skin at a final concentration of 0%, 0.001%, 0.01%, or 0.1%. Thereafter, culturing was performed in a 5%CO₂ incubator at 37°C for about 48 hours.

### (RNA extraction)

After carefully removing the medium and drugs in the well, the cells were washed 3 times with PBS. The culture cup was taken out from the well, the membrane was peeled off, and then the skin was transferred to a 10 cm dish (430167, manufactured by Corning Incorporated), cut into 1/2 with a high stainless double-edged razor, and further divided into the epidermis and the dermis.

The tissue piece of dermis was transferred to a 1.5 mL tube containing 300 µL of RLT solution (prepared with 2-mercaptetanol, 99% at 1% relative to Buffer RLT Lysis buffer), and thereafter RNA was extracted according to the protocol of RNeasy Fibrous Tissue Kit (74704, manufactured by QIAGEN). Specifically, after crushing the tissue piece of dermis, 590 µL of RNase free water and 10 µL of proteinaseK were added to perform proteinaseK treatment, followed by incubation at 55°C for 10 minutes. After centrifugation at 10,000 × g for 3 min, 450 µL of 99.5% EtOH was mixed. After transferring to a spin column and centrifuging, 350 µL of RW buffer was added, followed by centrifugation again. Next, a mixed solution of 10 µL of DNaseI solution dissolved in RNase free water and 70 µL of buffer RDD was added from the membrane, followed by incubation at room temperature for 15 minutes. Thereafter, 350 µL of RW buffer was added again, followed by centrifugation at 10,000 × g for 1 minute. To the mixture was added 500 µL of RPE buffer, followed by centrifugation at 10,000 × g twice (1 minute for first time, 2 minutes for second time). Finally, the mixture was centrifuged again and then dried. The column was replaced, 35 µL of RNase free water was added to elute RNA, and RNA concentration was measured.

### (cDNA synthesis)

cDNA was synthesized according to the protocol of ReverTra Ace (registered trademark) qPCR RT Master Mix (FSQ-301, manufactured by Toyobo Co., Ltd.). Specifically, setting the carried amount to 300 ng, 2 µL of 4× DN Master Mix was added to 6 µL obtained by mixing a sample and RNase free water depending on the measured RNA concentration, followed by a Dnase reaction at 37°C-5 minutes and 4°C-hold. Subsequently, 2 µL of 5× RT Master Mix II was mixed, followed by a reverse transcription reaction (RT reaction) in a flow of 37°C-15 minutes, 50°C-5 minutes, 98°C-5 minutes, and 4°C-hold. Four-fold dilution was performed with RNase free water.

### (RT-PCR)

Real-Time PCR was performed basically according to a protocol of TaqMan (registered trademark) Gene Expression Assays (manufactured by Thermo Fisher Scientific). To each primer for COL1A1, COL3A1, and ELN, 5 µL of TaqMan fast Master Mix (2x), 0.5 µL of TaqMan Gene Expression Assay (20x), 3.5 µL of RNase free water, and 1 µL of cDNA were prepared. Then, the mixture was added to a 384 well plate. Real-Time PCR was performed with a Quant studio 3Flex system. PCR cycle conditions were set to 50°C-2 minutes, 95°C-20 seconds, 95°C-1 second, and 60°C-20 seconds as 45 cycles.

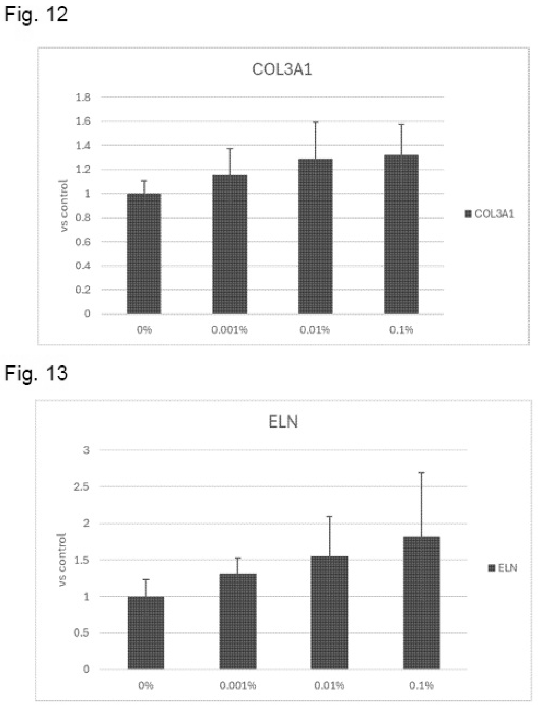

As shown in Figs. 11 to 13, it has been confirmed in the three-dimensional cultured skin that when the culture supernatant and/or extracellular vesicles of a microalga were applied to the three-dimensional cultured skin, the elastin gene expression, the COL1A1 gene expression, and the COL3A1 gene expression tended to be enhanced at a final concentration of 0.001% to 0.1% of the composition containing the extracellular vesicles (ultrafiltrated sample) derived from Pavlova sp. in a concentration-dependent manner.

## Claims

1. A composition for maintaining or increasing a fibrous structural protein, comprising a culture supernatant and/or extracellular vesicles of a microalga.

2. The composition according to claim 1, wherein the fibrous structural protein is collagen, elastin, or hyaluronic acid.

3. The composition according to claim 2, wherein the elastin is skin elastin.

4. The composition according to claim 1, wherein the maintenance or increase of the fibrous structural protein is due to suppression of a fibrous structural protein decomposition enzyme, activation of a fibrous structural protein synthase, or enhancement of gene expression of a fibrous structural protein.

5. The composition according to claim 4, wherein the fibrous structural protein decomposition enzyme is matrix metalloprotease-1.

6. A composition for maintaining or increasing skin elasticity, comprising a culture supernatant and/or extracellular vesicles of a microalga.

7. A composition for promoting production of collagen, elastin, or hyaluronic acid, comprising a culture supernatant and/or extracellular vesicles of a microalga.

8. The composition according to any one of claims 1 to 7, wherein the filter has a molecular weight cutoff of 10 KDa to 1000 KDa.

9. The composition according to any one of claims 1 to 7, wherein particle diameter of the extracellular vesicles is 50 nm to 700 nm.

10. The composition according to any one of claims 1 to 7, wherein a content of the culture supernatant and/or extracellular vesicles of a microalga derived from the microalga is 0.001 to 0.1 mass%.

11. The composition according to any one of claims 1 to 7, having a content of 1.0×10⁶ to 1.0×10¹¹ extracellular vesicles/mL derived from the microalga.

12. The composition according to any one of claims 1 to 7, wherein the microalga is a microalga belonging to a genus Pavlova, Euglena, Spirulina, or Chlorella.

13. The composition according to any one of claims 1 to 7, being a food or beverage product, a cosmetic product, a pharmaceutical product, or a quasi-pharmaceutical product.

14. The composition of any one of claims 1 to 7, wherein the composition is intended for young people.
